(19)

Europäisches Patentamt
European Patent Office
Office européen des brevets

(11) **EP 3 624 043 B1**

(12) **EUROPEAN PATENT SPECIFICATION**

(45) Date of publication and mention
of the grant of the patent:
**22.10.2025 Bulletin 2025/43**

(21) Application number: **18798937.1**

(22) Date of filing: **09.05.2018**

(51) International Patent Classification (IPC):
*G06Q 30/0251* (2023.01)    *G06Q 30/0601* (2023.01)
*G06Q 50/10* (2012.01)    *G06N 3/02* (2006.01)
*A61B 5/00* (2006.01)    *G06F 17/00* (2019.01)

(52) Cooperative Patent Classification (CPC):
**A61B 5/442; A61B 5/0077; G06N 3/02;
G06Q 30/0255; G06Q 30/0631; G06Q 50/10**

(86) International application number:
**PCT/KR2018/005121**

(87) International publication number:
**WO 2018/208044 (15.11.2018 Gazette 2018/46)**

(54) **METHOD AND APPARATUS FOR PROVIDING PERSONALIZED SKIN CARE GUIDE INFORMATION**

VERFAHREN UND VORRICHTUNG ZUR BEREITSTELLUNG VON PERSONALISIERTEN HAUTPFLEGEFÜHRUNGSINFORMATIONEN

PROCÉDÉ ET APPAREIL POUR FOURNIR DES INFORMATIONS PERSONNALISÉES DE GUIDE DE SOINS DE LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.05.2017  KR 20170058097**

(43) Date of publication of application:
**18.03.2020  Bulletin 2020/12**

(73) Proprietor: **Samsung Life Public Welfare
Foundation
Seoul 04348 (KR)**

(72) Inventor: **LEE, Jong Hee
Seoul 06648 (KR)**

(74) Representative: **Patentanwälte Olbricht Buchhold
Keulertz
Partnerschaft mbB
Neue Mainzer Straße 75
60311 Frankfurt am Main (DE)**

(56) References cited:
JP-A- 2006 142 005    KR-A- 20130 037 433
KR-A- 20150 120 542    KR-A- 20160 107 479
KR-A- 20170 007 030    US-A1- 2010 185 064

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 3 624 043 B1

## Description

### TECHNICAL FIELD

The present disclosure relates to a method and apparatus for providing personalized skin care guide information.

### BACKGROUND ART

The interest in skin care has recently increased regardless of age and gender, and the scale of the service industry specializing in skin care, the functional cosmetics industry for skin care, etc. has also increased day by day. As skin care-related services and products increase, consumers are faced with a variety of choices. Accordingly, consumers increasingly feel the need to accurately recognize their skin conditions and then select products/services suitable for the skin conditions.

However, unless the consumers are specialists, it is difficult to accurately recognize their skin conditions and it is expensive to find specialists and periodically check skin conditions. Accordingly, devices for measuring skin conditions which may be easily used even by the general public have recently emerged.

Korean Patent Publication Nos. 2015-0105574 and 2014-0075258 each disclose a system for interoperating with a mobile device or a communication device by measuring a skin condition through a separate skin condition measuring device or a skin scare device. US 2010/185064 A1 discloses a prior art computer system to provide skin care recommendations based on an image of a subject.

### DESCRIPTION OF EMBODIMENTS

### TECHNICAL PROBLEM

However, because such a skin care device has to be separately purchased, costs are incurred, and because the skin care device has to separately interoperate with a smartphone for tracking a skin condition, a user initially feels severe discomfort.

In addition, skin conditions of people are affected by an external environment such as a temperature, humidity, an ultraviolet index, or fine dust, a lifestyle such as a time when an outdoor activity is performed, a degree of gene expression, etc., and the skin care device fails to provide personalized information about which factor most affects a skin condition of each user.

Objectives of the present disclosure for solving various problems including the above problems are to provide a method and apparatus for providing personalized skin care guide information by analyzing a photograph of a subject's skin to obtain skin condition information, periodically collecting external environment data, lifestyle data, and skin condition index data, and analyzing a correlation between the data. However, such problems are merely examples, and the scope of the present disclosure is not limited by the problems.

### SOLUTION TO PROBLEM

The invention is defined by the appended claims. Preferred embodiments of the invention are defined by the dependent claims. A method of providing personalized skin care guide information, according to an embodiment of the present disclosure, includes obtaining external environment data, lifestyle data of a subject, and skin condition index data of the subject in intervals from a first interval to an $N^{th}$ interval; analyzing a correlation among the external environment data, the lifestyle data, and the skin condition index data in the intervals from the first interval to the $N^{th}$ interval; obtaining external environment data, lifestyle data of the subject, and skin condition index data of the subject in an $(N+1)^{th}$ interval; and providing skin care guide information of the subject to the subject, based on a result of the correlation analysis.

The external environment data may include at least one from among temperature, humidity, ultraviolet index, fine dust concentration, and ultrafine dust concentration data.

The obtaining of the external environment data may include: obtaining location information of the subject; and obtaining at least one from among temperature, humidity, ultraviolet index, fine dust concentration, and ultrafine dust concentration data corresponding to a location.

The lifestyle data includes at least one from among sleeping hours, alcohol consumption, skin care, an exercise time, an outdoor activity time, and a menstrual cycle.

The obtaining of the skin condition index data may include calculating a skin condition index by analyzing a photograph of the subject's skin.

The analyzing of the correlation includes training an artificial neural network by using the external environment data and the lifestyle data in the intervals from the first interval to the $N^{th}$ interval as input data for training and the skin condition index data as output data for training.

The providing of the skin scare guide information to the subject may include providing an alarm to the subject when a rate of change of a skin condition index of the $N^{th}$ interval and a skin condition index of the $(N+1)^{th}$ interval is equal to or greater than a threshold value.

The skin care guide information includes the cause of change information of the skin condition index data of the $N^{th}$ interval and the $(N+1)^{th}$ interval.

The skin care guide information may include at least one of guide information about usage of cosmetics, a lifestyle, nutrient intake, and cosmetic ingredients.

The providing of the skin care guide information may include: obtaining external environment data of an $(N+2)^{th}$ interval, in the $(N+1)^{th}$ interval; and providing skin care guide information of the $(N+2)^{th}$ interval, based on the result of the correlation analysis.

The method includes, before the providing of the skin care guide information, obtaining skin gene expression data of the subject.

An apparatus for providing personalized skin care guide information according to another embodiment of the present disclosure includes a data obtainer configured to obtain external environment data, lifestyle data of a subject, and skin condition index data of the subject in intervals from a first interval to an $N^{th}$ interval; a data analyzer configured to analyze a correlation among the external environment data, the lifestyle data, and the skin condition index data in the intervals from the first interval to the $N^{th}$ interval; and a skin care guide information provider configured to provide skin care guide information to the subject, based on a result of the correlation analysis, wherein the data obtainer is further configured to further obtain external environment data, lifestyle data of the subject, and skin condition index data of the subject in an $(N+1)^{th}$ interval.

The external environment data may include at least one from among temperature, humidity, ultraviolet index, fine dust concentration, and ultrafine dust concentration data.

The data obtainer may include a location information obtainer configured to obtain location information of the subject; and a weather information obtainer configured to obtain at least one from among temperature, humidity, ultraviolet index, and fine dust concentration data corresponding to a location.

The lifestyle data includes at least one from among sleeping hours, alcohol consumption, skin care, an exercise time, an outdoor activity time, and a menstrual cycle.

The data obtainer may include a skin condition index calculator configured to calculate a skin condition index by analyzing a photo of the subject's skin.

The data analyzer is further configured to train an artificial neural network by using the external environment data and the lifestyle data in the intervals from the first interval to the $N^{th}$ interval as input data for training and the skin condition index data as output data for training.

The skin care guide information provider may be further configured to provide an alarm to the subject when a rate of change of a skin condition index of the $N^{th}$ interval and a skin condition index of the $(N+1)^{th}$ interval is equal to or greater than a threshold value.

The skin care guide information includes the cause of change information of the skin condition index data of the $N^{th}$ interval and the $(N+1)^{th}$ interval.

The skin care guide information includes at least one of guide information about usage of cosmetics, a lifestyle, nutrient intake, and cosmetic ingredients.

The skin care guide information provider may be further configured to obtain external environment data of an $(N+2)^{th}$ interval, in the $(N+1)^{th}$ interval and provide skin care guide information of the $(N+2)^{th}$ interval, based on the result of the correlation analysis.

The data obtainer is further configured to obtain skin gene expression data of the subject.

Other aspects, features, and advantages of the present disclosure will become more apparent from the drawings, the claims, and the detailed description.

ADVANTAGEOUS EFFECTS OF DISCLOSURE

As described above, according to an embodiment of the present disclosure, there may be provided a method and apparatus for providing skin care guide information by analyzing in a personalized manner a correlation among an external environment, a lifestyle of a subject, and a skin condition of the subject.

Accordingly, a user may obtain personalized skin care guide information such as which factor of the external environment needs to be noted or which habit of the lifestyle needs to be improved.

BRIEF DESCRIPTION OF DRAWINGS

FIG. 1 is a diagram illustrating a configuration of an apparatus for providing personalized skin care guide information, according to an embodiment of the present disclosure.

FIG. 2 is a flowchart illustrating a method of providing personalized skin care guide information, according to an embodiment of the present disclosure, and FIG. 3 is an image illustrating the method of FIG. 2 over time.

FIG. 4 is an image illustrating steps of obtaining external environment data according to an embodiment.

FIG. 5 is an image illustrating a state in which a skin condition index of a subject is obtained from a photograph of the subject's skin.

FIG. 6 is a diagram schematically illustrating a topology of an artificial neural network used in a step of analyzing a correlation according to an embodiment of the present disclosure.

FIG. 7 illustrates graphs each showing a change in a skin condition index according to a change in an external environment.

FIG. 8 is an image illustrating a method of providing personalized skin care guide information, according to an embodiment.

FIG. 9 is an image illustrating a method of providing personalized skin care guide information, according to another embodiment.

FIG. 10 is a flowchart illustrating a method of providing personalized skin care guide information, according to another embodiment of the present disclosure.

BEST MODE

The present disclosure may include various embodiments and modifications, and embodiments thereof will be illustrated in the drawings and will be described herein in detail. The effects and features of the present disclosure and the accompanying methods thereof will become apparent from the following description of the embodiments, taken in conjunction with the accompanying drawings. However, the present disclosure is not limited to the embodiments described below, and may be embodied in various modes.

It will be understood that although the terms "first," "second," etc. may be used herein to describe various elements, these elements should not be limited by these terms and these terms are only used to distinguish one element from another.

As used herein, the singular forms "a," "an," and "the" are intended to include the plural forms as well, unless the context clearly indicates otherwise.

It will be further understood that the terms "comprises" and/or "comprising" used herein specify the presence of stated features or components, but do not preclude the presence or addition of one or more other features or components.

When a certain embodiment may be implemented differently, a specific process order may be different from the described order. For example, two consecutively described processes may be performed substantially at the same time or performed in an order opposite to the described order.

It will be further understood that the terms "comprises" and/or "comprising" specify the presence of stated features or components, but do not preclude the presence or addition of one or more other features or components.

Reference will now be made in detail to embodiments, examples of which are illustrated in the accompanying drawings, and in the drawings, the same elements are denoted by the same reference numerals and a repeated explanation thereof will not be given.

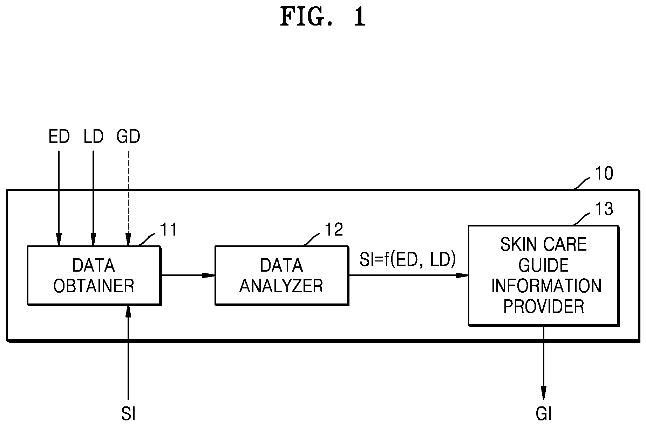

FIG. 1 is a diagram illustrating a configuration of an apparatus 10 for providing personalized skin care guide information, according to an embodiment of the present disclosure.

Only elements of the apparatus 10 related to the present embodiment are illustrated in FIG. 1 to prevent the features of the present embodiment from being obscure. Accordingly, it will be understood by one of ordinary skill in the art that the apparatus 10 may further include other general-purpose elements in addition to the elements illustrated in FIG. 1.

The apparatus 10 according to the present embodiment may correspond to at least one processor or may include at least one processor. Accordingly, the apparatus 10 may be included in another hardware apparatus such as a microprocessor or a general-purpose computer system.

The present disclosure may be described in terms of functional block components and various processing steps S. Such functional blocks may be realized by any number of hardware and/or software components configured to perform specified functions. For example, the present disclosure may employ various integrated circuit (IC) components, e.g., memory elements, processing elements, logic elements, and look-up tables, which may carry out a variety of functions under the control of one or more microprocessors or other control devices. Similarly, where the elements of the present disclosure are implemented using software programming or software elements, the disclosure may be implemented with any programming or scripting language such as C, C++, Java, assembler language, or the like, with various algorithms being implemented with any combination of data structures, objects, processes, routines, or other programming elements. Functional aspects may be implemented in algorithms that are executed on one or more processors. Furthermore, the present disclosure may employ any number of conventional techniques for electronics configuration, signal processing, and/or data processing. The words "mechanism," "element," "means," and "configuration" are used broadly and are not limited to mechanical or physical embodiments. The terms may include software routines in conjunction with processors, etc.

Referring to FIG. 1, the apparatus 10 includes a data obtainer 11, a data analyzer 12, and a skin care guide information provider 13.

The data obtainer 11 obtains external environment data ED, lifestyle data LD of a subject, and skin condition index data SI. The data obtainer 11 may selectively obtain skin gene expression data GD of the subject, which will be described below.

The external environment data ED may include various weather information that may affect a skin condition and may include at least one from among, but not limited to, temperature, humidity, ultraviolet index, fine dust concentration, and ultrafine dust concentration data.

The data obtainer 11 may obtain the external environment data ED from an external database (DB) such as a meteorological office. Alternatively, the data obtainer 11 may directly obtain the external environment data ED from a temperature sensor, a humidity sensor, an ultraviolet sensor, a fine dust sensor, etc. connected to the apparatus 10.

The lifestyle data LD includes information about a lifestyle that may affect the skin condition of the subject and may include at least one from among, but not limited to, sleeping hours, alcohol consumption, skin care, an exercise time, an outdoor activity time, and a menstrual cycle.

The data obtainer 11 may directly obtain the lifestyle data LD from the subject. That is, when the subject inputs a value corresponding to each item through an input unit, the data obtainer 11 connected to the input unit may obtain the value. Alternatively, the data obtainer 11 may directly obtain all or some of the external environment data ED by estimating sleeping hours, an exercise time, and an outdoor activity time from a global positioning system (GPS) sensor, an acceleration sensor, etc. connected to the apparatus 10.

According to an embodiment, the data obtainer 11 may include a location information obtainer that obtains location information of the subject and a weather information obtainer that obtains at least one from among temperature, humidity, ultraviolet index, and fine dust concentration data corresponding to a location of the subject.

The skin condition index data SI refers to data obtained by analyzing and scoring the skin condition of the subject. The skin condition index data SI may include, for example, a wrinkle index, a pigment index, and a trouble index respectively indicating a degree of wrinkles, a degree of pigmentation, and a degree of trouble. Alternatively, the skin condition index data SI may be an average value of each index.

The data obtainer 11 may include a skin condition index calculator that calculates a skin condition index by analyzing a photograph of the subject's skin. In this case, the data obtainer 11 may directly obtain the skin condition index data SI from the photograph of the subject.

In this case, each of the external environment data ED, the lifestyle data LD, and the skin condition index data SI may be obtained for each interval. In the present disclosure, the interval may be, but is not limited to, 24 hours, 48 hours, 72 hours, or a week. For example, when the interval is 24 hours, the external environment data ED, the lifestyle data LD, and the skin condition index data SI may be repeatedly obtained on a daily basis.

The data analyzer 12 analyzes a correlation among the external environment data ED, the lifestyle data LD, and the skin condition index data SI. In this case, analyzing the correlation may mean analyzing how the skin condition index is affected by an external environment and a lifestyle, and may mean calculating an approximation formula of a function $SI = f(ED, LD)$ by using the external environment data ED and the lifestyle data LD as an input and the skin condition index data SI as an output.

The data analyzer 12 analyzes the correlation among the data based on data in intervals from a first interval to an $N^{th}$ interval. The skin care guide information provider 13 provides skin care guide information GI to the subject based on a result of the correlation analysis. It is preferable that the correlation analysis is completed before the skin care guide information provider 13 provides the skin care guide information GI to the subject in an $(N+1)^{th}$ interval.

In summary, for example, the data obtainer 11 accumulates the external environment data ED, the lifestyle data LD, and the skin condition index data SI for a first day to an $N^{th}$ day. The data analyzer 12 analyzes a correlation among the data based on the accumulated data. On an $(N+1)^{th}$ day, new data is accumulated, and the skin care guide information provider 13 analyzes a cause of change in the skin condition index data SI of the $(N+1)^{th}$ day based on a result of the correlation analysis to the $N^{th}$ day and provides the guide information GI to the subject.

In this case, because the present disclosure is inductively defined, data acquisition and correlation analysis results are updated as N increases. N is a natural number, and when N is 1, the expression 'intervals from a first interval to an $N^{th}$ interval' may mean 'a first interval'.

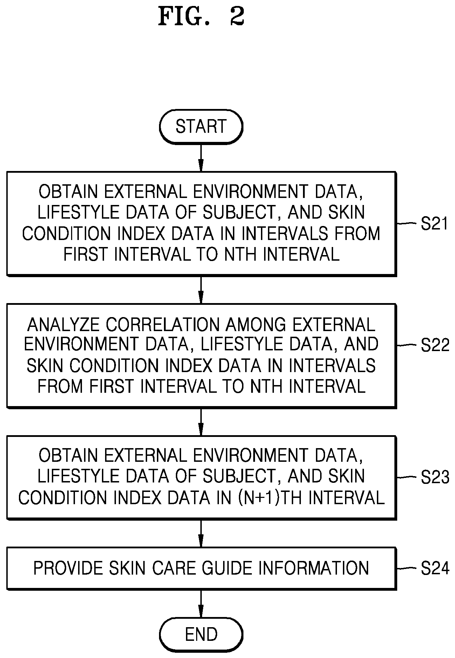

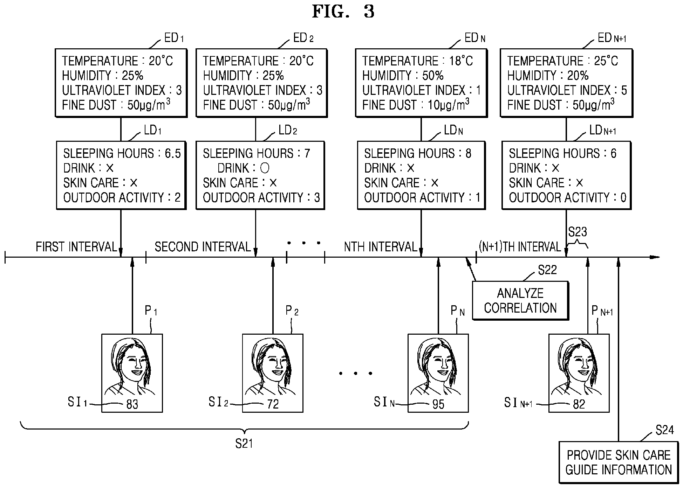

FIG. 2 is a flowchart illustrating a method of providing personalized skin care guide information, according to an embodiment of the present disclosure, and FIG. 3 is an image illustrating the method of FIG. 2 over time.

The method of providing personalized skin care guide information, according to an embodiment of the present disclosure, includes a step S21 of obtaining external environment data $ED_1 \sim ED_N$, lifestyle data $LD_1 \sim LD_N$ of a subject, and skin condition index data $SI_1 \sim SI_N$ in intervals from a first interval to an $N^{th}$ interval; a step S22 of analyzing a correlation among the external environment data $ED_1 \sim ED_N$, the lifestyle data $LD_1 \sim LD_N$, and the skin condition index data $SI_1 \sim SI_N$ in the intervals from the first interval to the $N^{th}$ interval; a step S23 of external environment data $ED_{N+1}$, lifestyle data $LD_{N+1}$ of the subject, and skin condition index data $SI_{N+1}$ of the subject in an $(N+1)^{th}$ interval; and a step S24 of providing skin care guide information to the subject based on a result of the correlation analysis.

Referring to FIGS. 2 and 3, the step S21 of obtaining the external environment data $ED_1 \sim ED_N$, the lifestyle data $LD_1 \sim LD_N$ of the subject, and the skin condition index data $SI_1 \sim SI_N$ of the subject in the intervals from the first interval to the $N^{th}$ interval is performed.

For convenience of explanation, the following will be described on the assumption that each interval is a day.

First, the external environment data $ED_1$ and the lifestyle data $LD_1$ of a first day are obtained. In this case, the first day may be a day when the method or apparatus of the present disclosure is first used. Although examples of the external environment data $ED_1$ include a temperature, humidity, an ultraviolet index, and fine dust and examples of the lifestyle data $LD_1$ include sleeping hours, alcohol consumption, skin care, and an outdoor activity time in FIG. 3, the present disclosure is not limited thereto. Although the external environment data $ED_1$ and the lifestyle data $LD_1$ are simultaneously obtained in FIG. 3, the external environment data $ED_1$ and the lifestyle data $LD_1$ may be obtained at different times.

Next, the skin condition index data $SI_1$ of the first day is obtained. In this case, the skin condition index data $SI_1$ may be obtained by analyzing a photograph $P_1$ of the subject's skin on the first day. Unlike in FIG. 3, a time when the skin condition index data $SI_1$ is obtained may be earlier than a time when the external environment data $ED_1$ and the lifestyle data $LD_1$ are obtained.

The external environment data $ED_2$ and the lifestyle data $LD_2$ of the next day, that is, a second day, are obtained. Due to a change in an external environment and a lifestyle that affect a skin condition, a skin condition index may have a value different from that of the first day. Next, the skin condition index data $SI_2$ of the second day is obtained. In this case, the skin condition index data $SI_2$ may be obtained by analyzing a photograph $P_2$ of the subject's skin on the second day.

Such data collection is repeatedly performed every day, and thus the external environment data $ED_N$, the lifestyle data $LD_N$, and the skin condition index data $SI_N$ obtained by analyzing an $N^{th}$ photograph $P_N$ are obtained on an $N^{th}$ day. Accordingly, the step S21 of obtaining the external environment data $ED_1 \sim ED_N$, the lifestyle data $LD_1 \sim LD_N$, and the skin condition index data $SI_1 \sim SI_N$ during days from the first day to the $N^{th}$ day is performed. When the subject does not input the lifestyle data LD or does not take a photograph on one of the days and thus the lifestyle data LD and the skin condition index data SI are not obtained, the day may be excluded from correlation analysis.

Next, the step of S22 of analyzing the correlation among the external environment data $ED_1 \sim ED_N$, the lifestyle data $LD_1 \sim LD_N$, and the skin condition index data $SI_1 \sim SI_N$ in the intervals from the first interval to the $N^{th}$ interval is performed.

Because the external environment, the lifestyle, and the skin condition index are changed every day, some data has to be accumulated in order to analyze a correlation among the external environment data ED, the lifestyle data LD, and the skin condition index data SI. That is, for statistically meaningful correlation analysis, a certain period of time has to pass, and the accuracy of a correlation analysis result may increase as N increases.

In this case, analyzing the correlation may mean analyzing how the skin condition index is affected by the external environment and the lifestyle. For example, through correlation analysis, whether the subject is sensitive to ultraviolet light, fine dust, or sleeping hours may be determined. The sensitivity may be labeled as high/medium/low.

Next, the step S23 of obtaining the external environment data $ED_{N+1}$, the lifestyle data $LD_{N+1}$ of the subject, and the skin condition index data $SI_{N+1}$ obtained by analyzing a photograph $P_{N+1}$ of the subject's skin in the $(N+1)^{th}$ interval and the step S24 of providing the skin care guide information to the subject based on the result of the correlation analysis are performed.

Because the correlation analysis of the skin condition according to the external environment and the lifestyle is performed on the $N^{th}$ day, a cause of change in the skin condition index of an $(N+1)^{th}$ day may be analyzed and may be guided to the subject. For example, it is assumed that as an analysis result of the external environment data ED, the lifestyle data LD, and the skin condition index data SI of the subject during the days from the first day to the $N^{th}$ day, the subject belongs to an ultraviolet sensitive group. When the ultraviolet index increased and the skin condition index $SI_{N+1}$ decreased on the $(N+1)^{th}$ day, it may be notified to the subject that the skin condition index decreased due to the increase in the ultraviolet index.

That is, after data accumulation and analysis is performed during the days from the first day to the $N^{th}$ day, personalized skin care guide information including information such as which factor of the external environment needs to be noted or which habit of the lifestyle needs to be improved on the $(N+1)^{th}$ day may be provided.

When time passes and it is an $(N+2)^{th}$ day, correlation analysis including the external environment data $ED_1 \sim ED_{N+1}$, the lifestyle data $LD_1 \sim LD_{N+1}$, and the skin condition index data $SI_1 \sim SI_{N+1}$ in intervals from the first interval to the $(N+1)^{th}$ interval is performed again. That is, when time passes and N increases, a correlation analysis result is continuously updated. In this case, because data closer to a time when skin care guide information is provided is more important, a greater weight may be applied to data closer to a current time during correlation analysis. Alternatively, only the external environment data ED, the lifestyle data LD, and the skin condition index data SI 1 month, 3 months, 6 months, or 12 months before the time when the skin care guide information is provided may be used for the correlation analysis.

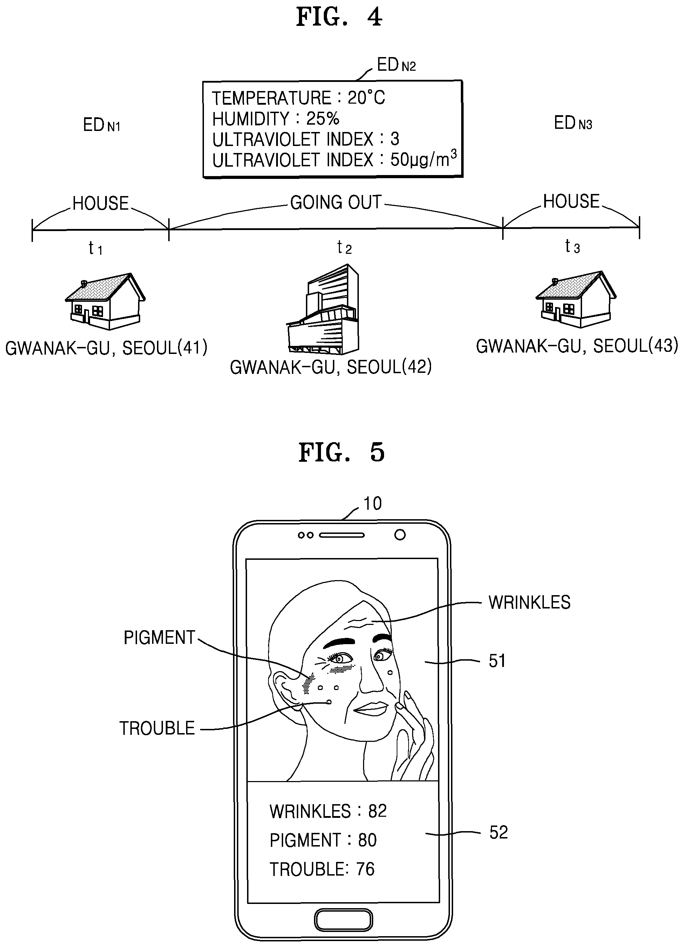

FIG. 4 is an image illustrating the steps of S21 and S23 of obtaining the external environment data ED according to an embodiment.

According to an embodiment, the steps S21 and S23 of obtaining the external environment data ED may include a step of obtaining location information of a subject; and a step of obtaining at least one from among temperature, humidity, ultraviolet index, fine dust concentration, and ultrafine dust concentration data corresponding to a location.

An objective of the present disclosure is to analyze in a personalized manner a correlation among an external environment, a lifestyle of the subject, and a skin condition of the subject and provide skin care guide information. Accordingly, it is preferable that the external environment data ED contains as much information as possible about the external environment to which the subject is actually exposed.

Referring to FIG. 4, a daily life pattern of the subject who returns on after going out in the afternoon is schematically illustrated. In this case, when the location information of the subject is obtained and it is recognized that the subject is located at substantially the same location from the evening to the morning, the subject may be assumed to be in his/her 'house'.

In particular, external environment factors such as an ultraviolet index and a fine dust concentration affect a skin condition of the subject when he/she 'goes out'. Accordingly, when the location information of the subject is obtained and it is recognized that the subject is outside the 'house', it is preferable that external environment data $ED_{N2}$ of a location where the subject 'goes out' is obtained. Accordingly, in this case, a value of each item of the external environment data ED may be calculated considering a time when the subject is in the house and a time when the subject goes out.

For example, it is assumed that a fine dust concentration and a humidity of external environment data $ED_{N1}$ during a morning time $t_1$ in Gwanak-gu, Seoul where the subject lives are $a_1$ and $h_1$, a fine dust concentration and a humidity of external environment data $ED_{N3}$ during an evening time $t_3$ are $a_3$ and $h_3$ on average, and a fine dust concentration and a humidity of the external environment data $ED_{N2}$ during a day time $t_2$ are $a_2$ and $h_2$. In this case, because the subject may be exposed to fine dust when the subject goes out, a value of a 'fine dust concentration' from among items of the external environment data ED may be modified as shown in <Equation 1>.

<Equation 1>

$$\text{Fine dust concentration} = a_2 * t_2 / (t_1 + t_2 + t_3)$$

Because a difference between a humidity inside the house and a humidity outside the house may not be large, a value of a 'humidity' from among the items of the external environment data ED may be modified as shown in <Equation 2>.

<Equation 2>

$$\text{Humidity} = (h_1 * t_1 + h_2 * t_2 + h_3 * t_3) / (t_1 + t_2 + t_3)$$

That is, a result obtained after analyzing the life pattern of the subject using the location information may be used for correlation analysis between the external environment data ED and the skin condition index data SI.

A time during which the subject 'goes out' may be directly input to an input unit by the subject and may be obtained by the data obtainer 11 connected to the input unit. That is, for example, when the subject inputs a 'going out' time excluding a time during which the subject is in a building such as the house/school/office, a weight proportional to the going out time may be applied to the external environment data ED.

FIG. 5 is an image illustrating a state where a skin condition index of a subject is obtained from a photograph of the subject's skin. In FIG. 5, for convenience of explanation, the apparatus 10 is embedded in a smartphone.

According to an embodiment, the steps S21 and S23 of obtaining the skin condition index data SI include a step of calculating a skin condition index by analyzing a photograph of the subject's skin.

Referring to FIG. 5, the apparatus 10 calculates a skin condition index 52 by analyzing a photograph 51 of the subject's skin. In an embodiment of the present disclosure, wrinkles, pigmentation, and trouble shown on the photograph of the subject's skin, particularly, the subject's face, are scored by using a method of quantizing a skin condition.

A skin condition index calculator included in the data obtainer 11 may obtain a wrinkle index by measuring a smoothness of skin pixels of the surface of the region under the eye, the forehead, or the like and scoring the smoothness (e.g., 0-100). The wrinkle index may be used as an indicator for checking a skin hydration state.

The skin condition index calculator may obtain a pigment index by scoring how wide/how many areas of pixels are represented in brown on the skin surface.

The skin condition index calculator may obtain a trouble index by scoring how wide/how many areas of pixels with a large roughness are represented in red colors on the skin surface. The trouble index is used as an index for reflecting skin trouble such as acne and rashes.

In this case, the apparatus 10 may be embedded in a mobile smartphone including a camera. In this case, when the subject takes a photograph of his/her skin by using the camera included in the mobile smartphone, the apparatus 10 analyzes the photograph and calculates a skin condition index. Accordingly, because skin condition information may be recognized without a separate skin condition measuring device, user convenience is improved.

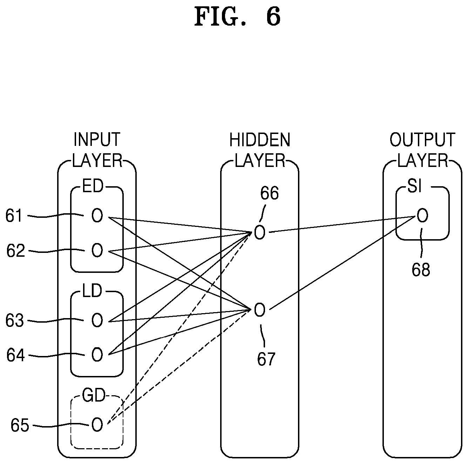

FIG. 6 is a diagram schematically illustrating a topology of an artificial neural network used in the step S23 of analyzing a correlation according to an embodiment of the present disclosure.

According to an embodiment, the step S23 of analyzing a correlation includes a step of training an artificial neural network by using the external environment data $ED_1 \sim ED_N$ and the lifestyle data $LD_1 \sim LD_N$ as input data for training and the skin condition index data $SI_1 \sim SI_N$ as an output data for training in intervals from a first interval to an $N^{th}$ interval.

The input data for training refers to data to be input to a node of an input layer to train the artificial neural network. In this case, the input data for training includes the external environment data ED and the lifestyle data LD, and includes the skin gene expression data GD which will be described below.

The output data for training refers to data to be compared with a value of a node output to an output layer to train the artificial neural network. The output data for training is the skin condition index data SI. The input data for training and the output data for training correspond to each other in each interval. That is, the output data for training corresponding to the external environment data $ED_N$ and the lifestyle data $LD_N$ that are the input data for training of the $N^{th}$ interval becomes the skin condition index data $SI_N$ of the $N^{th}$ interval. However, the same skin gene expression data GD is used as the input data for training, regardless of intervals.

The artificial neural network includes the input layer including a plurality of nodes and at least one hidden layer and output layer.

The 'input layer' refers to a set of nodes having specific variables assigned by a user, the 'output layer' refers to a set of nodes having results of a procedure according to the procedure set by the user, and the 'hidden layer' refers to a set of nodes that store intermediate results and temporary values that temporarily appear when the procedure set by the user is performed. The 'node' refers to an abstract object having a specific value that may be changed through a specific algorithm and connectable to another node.

Links may exist between the nodes of the input layer and the nodes of the hidden layer, and between the nodes of the hidden layer and the nodes of the output layer, and each of the links may have a specific weight assigned by the procedure set by the user.

The input layer includes nodes 61 and 62 to which items of the external environment data ED are input, and nodes 63 and 64 to which items of the lifestyle data LD are input. Although two nodes of the input layer are assigned to each of the external environment data ED and the lifestyle data LD in FIG. 6 for convenience of explanation, the present disclosure is not limited thereto. The input layer may include a node 65 to which the gene expression data GD is input.

A value of a node of the output layer output through an activation function and a weighted output of each node connection may be compared with a value of the output data for training. Although the output layer may include three nodes such as [a wrinkle index node, a pigment index node, and a trouble index node], the output layer is shown to include one node 68 in FIG. 6 for convenience of explanation.

The hidden layer connects the input layer where the input data for training obtained from the external environment data ED and the lifestyle data LD is input to the output layer including a 'skin condition index node'. Although the hidden layer is a single layer in FIG. 6 for convenience of explanation, the hidden layer may include a plurality of layers. Nodes of each hidden layer may be fully connected nodes of another adjacent hidden layer.

When each input data for training is input to the input layer and is output through the hidden layer to the output layer, the artificial neural network is trained by adjusting a weight of each node connection to minimize a difference between an output value (predicted value) and a value (actual value) of each output data for training corresponding to each input data for training.

A weight of a node connection of the input layer indicates a correlation among the external environment data ED, the lifestyle data LD, and a skin condition index. When a weight of a specific node connection of the input layer is large, an item corresponding to the specific node may be interpreted as greatly affecting a skin condition.

When the artificial neural network is trained in this way, it may accurately analyze which factor from among the external environment data ED and the lifestyle data LD particularly affects the skin condition for each individual.

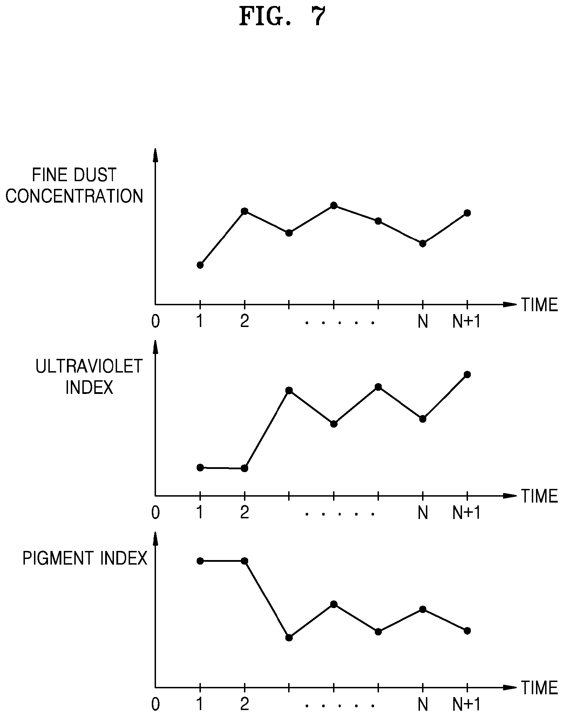

FIG. 7 illustrates graphs each showing a change in a skin condition index according to a change in an external environment. The graphs of FIG. 7 respectively show changes in a fine dust concentration and an ultraviolet index from among the external environment data ED and a pigment index from among the skin condition index data SI of a specific subject A over time.

It is known that fine dust is absorbed through skin to cause skin aging, lack of moisture, wrinkles, and pigmentation, and ultraviolet light produces melanin to cause pigmentation such as blemishes and freckles. The pigment index is an index indicating a degree of pigmentation, and decreases as pigmentation increases.

In this case, the ultraviolet index graph and the pigment index graph of the subject A appear to be vertically symmetric to each other. Accordingly, it may be analyzed that the ultraviolet index and the pigment index of the subject A are negatively correlated with each other. That is, it may be found that as ultraviolet light increases and the ultraviolet index increases, pigmentation increases and the pigment index of the subject A decreases, whereas, as the ultraviolet index decreases, pigmentation decreases and the pigment index of the subject A increases.

However, there is no large correlation between the fine dust concentration and the pigment index of the subject A.

That is, when a correlation between the external environment data $ED_1 \sim ED_N$ and the skin condition index data $SI_1 \sim SI_N$ in intervals from a first interval to an $N^{th}$ interval is analyzed, it may be derived that the subject A's skin is sensitive to ultraviolet light but is less sensitive to fine dust.

In this case, even when both the fine dust concentration and the ultraviolet index, which generally affect pigmentation increase in an $(N+1)^{th}$ interval, it is preferable that guide information about the 'ultraviolet index' is mainly provided to the subject A. This will be described below.

In the above example, a simple situation is exemplarily described for convenience of explanation, and a correlation analysis result of the present disclosure may include various information.

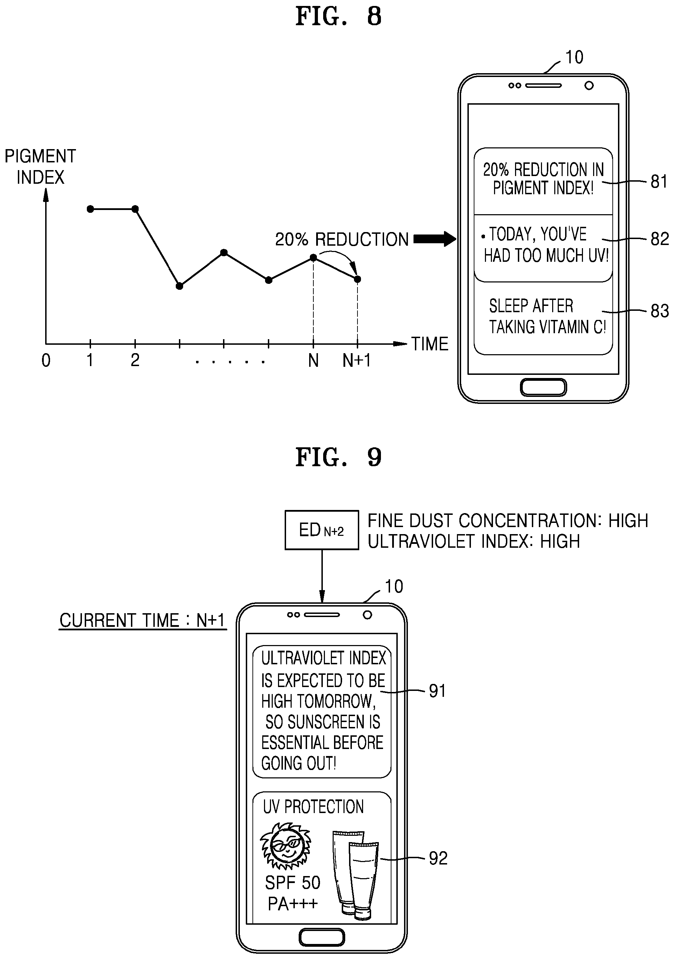

FIG. 8 is an image illustrating a method of providing personalized skin care guide information, according to an embodiment. In FIG. 8, for convenience of explanation, the apparatus 10 is embedded in a smartphone.

According to an embodiment, the step of S24 of providing skin care guide information to a subject may further include a step of providing an alarm to the subject when a rate of change of the skin condition index $SI_N$ of an $N^{th}$ interval and the skin condition index $SI_{N+1}$ of an $(N+1)^{th}$ interval is equal to or greater than a threshold value.

Referring to FIG. 8, a pigment index graph in intervals from a first interval to the $(N+1)^{th}$ interval is illustrated, like in FIG. 7. It is assumed that the result that, before data of the $(N+1)^{th}$ interval is obtained, a correlation between the external environment data $ED_1 \sim ED_N$ and the skin condition index data $SI_1 \sim SI_N$ in the intervals from the first interval to the $N^{th}$ interval is analyzed and the subject A's skin is sensitive to ultraviolet light but is less sensitive to fine dust is derived.

In this case, when a rate of change $((SI_{N+1}-SI_N)/SI_N)$ of a skin condition index is equal to or greater than a threshold value, the apparatus 10 may command a display unit to display an alarm 81 for notifying that the rate of change is equal to or greater than the threshold value. The threshold value may be equal to or greater than, for example, about 15%, and may be set by the subject.

When the rate of change of the skin condition index is equal to or greater than the threshold value according to an embodiment, whether an $(N+1)^{th}$ photograph is taken in a state similar to a state where first to $N^{th}$ photographs are taken may be checked by the subject. Because the skin condition index is an index measured based on a photograph analysis result, it is preferable that a time and a place where a photograph is taken are maintained. If a photographing place is changed due to a trip or the like, the consistency of data acquisition may be maintained when a photograph is taken in an environment having brightness that is similar to that in a house.

When it is determined that the subject takes the $(N+1)^{th}$ photograph in a state not similar to a state where the first photograph to the $N^{th}$ photograph are taken, the apparatus 10 may command the display unit to 'display a message indicating that a photograph is to be taken again in a similar state'. Next, when the subject takes a photograph again, a cause of change in the skin condition index may be analyzed by using the photograph as the $(N+1)^{th}$ photograph.

According to an embodiment, the skin care guide information may include the cause of change information of the skin condition index data $SI_N$ and $SI_{N+1}$ of the $N^{th}$ interval and the $(N+1)^{th}$ interval. The cause of change information refers to information about which factor from among items included in the external environment data ED and the lifestyle data LD causes a change in a skin condition. For example, the apparatus 10 may command a display unit of a mobile smartphone as shown in FIG. 8 to display content indicating that a pigment index is reduced due to ultraviolet light.

When a change in the skin condition index data SI relates to a lifestyle such as sleeping hours or an exercise time, the subject may obtain feedback on his/her lifestyle. When a change in the skin condition index data SI relates to an external environment such as an ultraviolet index, the subject may pay attention to the external environment to which he/she is exposed. Accordingly, the subject may obtain useful information for improving his/her skin condition.

According to an embodiment, the skin care guide information may include at least one of guide information about usage of cosmetics, a lifestyle, nutrient intake, and cosmetic ingredients.

Referring to reference numeral 83 of FIG. 8, guide information about specific nutrient intake is provided to the subject A whose pigment index is reduced because he/she is exposed to ultraviolet light and pigmentation occurs. Accordingly, the subject obtains the skin care guide information suitable for his/her condition.

MODE OF DISCLOSURE

FIG. 9 is an image illustrating a method of providing personalized skin care guide information, according to another embodiment. In FIG. 9, for convenience of explanation, the apparatus 10 is embedded in a smartphone.

According to an embodiment, the step of S24 of providing skin care guide information may further include a step of obtaining external environment data $ED_{N+2}$ of an $(N+2)^{th}$ interval, in the $(N+1)^{th}$ interval; and a step of S of providing skin care guide information of the $(N+2)^{th}$ interval based on a result of correlation analysis.

Referring to FIG. 9, a step of obtaining the external environment data ED of the $(N+2)^{th}$ interval, at the time of an

$(N+1)^{th}$ interval. For example, a weather forecast for 'tomorrow' that is an $(N+2)^{th}$ day may be obtained, at the time of 'today' that is an $(N+1)^{th}$ day. Next, skin care guide information 91 to be noted 'tomorrow' may be provided. That is, skin care guide information may include feedback information about a change in a skin condition 'today' and skin care guide information to be noted 'tomorrow'.

In this case, the skin care guide information of 'tomorrow' may be provided in a personalized manner. For example, it is assumed that it is predicted that both a fine dust concentration and an ultraviolet index 'tomorrow' in the $(N+2)^{th}$ interval are high as shown in FIG. 9. Because the subject A is more sensitive to ultraviolet light than fine dust as a result of correlation analysis, skin care guide information mainly indicating that the subject needs to be careful of ultraviolet light 'tomorrow' may be provided. The skin care guide information may also include advertisement information 92 about cosmetics to be recommended to the subject.

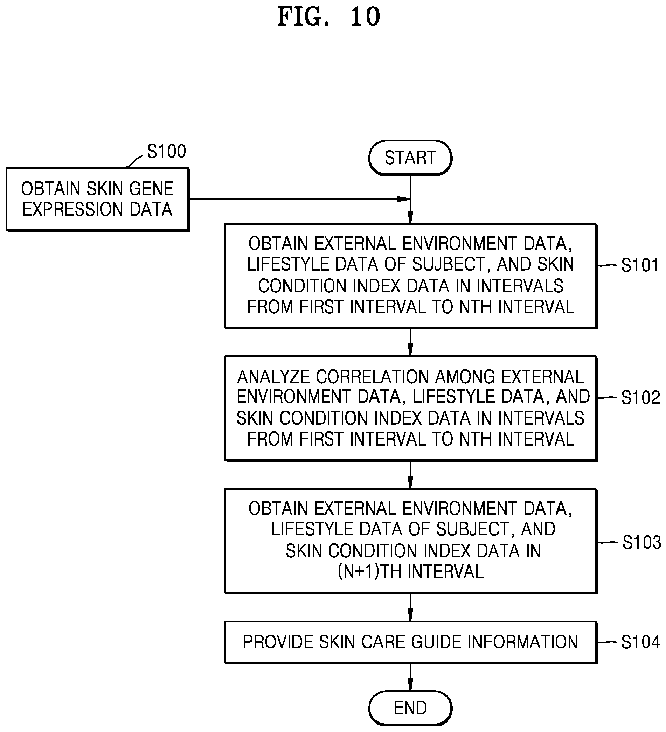

FIG. 10 is a flowchart illustrating a method of providing personalized skin care guide information, according to another embodiment of the present disclosure.

The method of providing personalized skin care guide information, according to an embodiment, further includes a step S100 of obtaining the skin gene expression data GD of a subject before a step S104 of providing skin care guide information.

The skin gene expression data GD (see FIG. 1) may be data about a gene affecting a skin condition such as a degree of skin hydration, a pigment, wrinkles, or an inflammation. In this case, for example, the skin gene expression data GD may be a value that classifies the amount of expression of each gene into high/medium/low levels. The skin gene expression data GD may be obtained by performing an oral mucosa rubbing test or a blood test.

Although the step S100 of obtaining the skin gene expression data GD is performed right before a step S101 of obtaining other data in FIG. 10, this is merely an example and the step S100 of obtaining the skin gene expression data GD may be performed at any time before the step S104 of providing skin care guide information. The step S100 does not need to be periodically performed, unlike when the external environment data ED, the lifestyle data LD, and the skin condition index data SI are obtained.

Because the skin gene expression data GD may be an index showing an innate skin condition of the subject, the skin gene expression data GD has a correlation with a skin condition index. For example, when the amount of gene expression affecting a degree of skin hydration belongs to a 'high' level, it may be expected that a skin wrinkle index that indicates a skin hydration state may be maintained constant regardless of a change in an external environment.

However, when the amount of expression in polymorphism analysis belongs to a high level as a result of a skin gene test, it does not necessarily mean that it affects the skin condition. Accordingly, even when the skin gene test is performed, accurate personalized skin care guide information may be provided only by mutually analyzing the external environment data ED, the lifestyle data LD, and the skin condition index data SI.

For example, it is assumed that a skin pigment index of a subject B having the skin gene expression data GD of [skin hydration: high, pigment: high, wrinkles: normal, and inflammation: low] is changed by about 20-25% whenever the subject B does an outdoor activity. In this case, it may be analyzed that although the subject B hardly produces pigments genetically, the subject B is sensitive to ultraviolet light and undergoes a severe change in pigments. Accordingly, skin care guide information indicating that the subject B needs to carefully apply antioxidant basic cosmetics before going out and apply a sunscreen every two hours may be provided to the subject B.

Referring back to FIG. 1, the data obtainer 11 may obtain the skin gene expression data GD of the subject. In this case, the data analyzer 12 may calculate an approximation formula $SI = f(ED, LD, GD)$ by using the external environment data ED, the lifestyle data LD, and the skin gene expression data GD as an input and the skin condition index data SI as an output.

While the present disclosure has been particularly shown and described with reference to embodiments thereof, it will be understood by one of ordinary skill in the art that various changes and equivalent other embodiments may be made therein. Accordingly, the spirit and scope of the present disclosure are defined by the following claims.

INDUSTRIAL APPLICABILITY

The present disclosure may be used in a method and apparatus for providing personalized skin care guide information.

**Claims**

1. A method of providing personalized skin care guide information, the method comprising:

   obtaining skin gene expression data of a subject;
   obtaining external environment data, lifestyle data of the subject, and skin condition index data of the subject in intervals from a first interval to an $N^{th}$ interval;

analyzing a correlation among the skin gene expression data, the external environment data, the lifestyle data, and the skin condition index data in the intervals from the first interval to the $N^{th}$ interval;

obtaining external environment data, lifestyle data of the subject, and skin condition index data of the subject in an $(N+1)^{th}$ interval; and

providing skin care guide information of the subject to the subject, based on a result of the correlation analysis, the lifestyle data includes at least one from among sleeping hours, alcohol consumption, skin care, an exercise time, an outdoor activity time, and a menstrual cycle,

the correlation is calculated using the external environment data and the lifestyle data as an input and the skin condition index data as an output, and

the skin care guide information of the subject includes a cause of change in the skin condition index data SI of the $(N+1)^{th}$ day based on a result of the correlation analysis to the $N^{th}$ day after obtaining external environment data, lifestyle data, and skin condition index data of the $(N+1)^{th}$ day, whereby the analyzing of the correlation comprises training an artificial neural network by using the external environment data and the lifestyle data in the intervals from the first interval to the Nth interval, the skin gene expression data as input data for training and the skin condition index data as output data for training.

2. The method of claim 1, wherein the external environment data comprises at least one from among temperature, humidity, ultraviolet index, fine dust concentration, and ultrafine dust concentration data.

3. The method of claim 2, wherein the obtaining of the external environment data comprises:

obtaining location information of the subject; and
obtaining at least one from among temperature, humidity, ultraviolet index, fine dust concentration, and ultrafine dust concentration data corresponding to a location.

4. The method of claim 1, wherein the lifestyle data comprises at least one from among an exercise time, and an outdoor activity time.

5. The method of claim 1, wherein the obtaining of the skin condition index data comprises calculating a skin condition index by analyzing a photograph of the subject's skin.

6. The method of claim 1, wherein the providing of the skin scare guide information to the subject comprises providing an alarm to the subject when a rate of change of a skin condition index of the $N^{th}$ interval and a skin condition index of the $(N+1)^{th}$ interval is equal to or greater than a threshold value.

7. The method of claim 1, wherein each of the external environment data, the lifestyle data and the skin condition index data in the correlation is assigned a weight and the weight is set to a larger value as the data obtained at a time closer to the current time is obtained.

8. The method of claim 1, wherein the skin care guide information comprises at least one of guide information about usage of cosmetics, a lifestyle, nutrient intake, and cosmetic ingredients.

9. The method of claim 1, wherein the providing of the skin care guide information comprises:

obtaining external environment data of an $(N+2)^{th}$ interval, in the $(N+1)^{th}$ interval; and
providing skin care guide information of the $(N+2)^{th}$ interval, based on the result of the correlation analysis.

10. An apparatus for providing personalized skin care guide information, the apparatus comprising:

a data obtainer configured to obtain skin gene expression data of a subject, external environment data, lifestyle data of a subject, and skin condition index data of the subject in intervals from a first interval to an $N^{th}$ interval;
a data analyzer configured to analyze a correlation among skin gene expression data, the external environment data, the lifestyle data, and the skin condition index data in the intervals from the first interval to the $N^{th}$ interval; and
a skin care guide information provider configured to provide skin care guide information to the subject, based on a result of the correlation analysis,
wherein the data analyzer is trained an artificial neural network by using the skin gene expression data, the external environment data and the lifestyle data in the intervals from the first interval to the Nth interval as input data for training and the skin condition index data as output data for training,

the data obtainer is further configured to further obtain external environment data, lifestyle data of the subject, and skin condition index data of the subject in an $(N+1)^{th}$ interval,

the lifestyle data includes at least one from among sleeping hours, alcohol consumption, skin care, an exercise time, an outdoor activity time, and a menstrual cycle,

the correlation is calculated using the external environment data and the lifestyle data as an input and the skin condition index data as an output, and

the skin care guide information of the subject includes a cause of change in the skin condition index data SI of the $(N+1)^{th}$ day based on a result of the correlation analysis to the $N^{th}$ day after obtaining external environment data, lifestyle data, and skin condition index data of the $(N+1)^{th}$ day.

11. The apparatus of claim 10, wherein the external environment data comprises at least one from among temperature, humidity, ultraviolet index, fine dust concentration, and ultrafine dust concentration data.

12. The apparatus of claim 10, wherein the data obtainer comprises:

a location information obtainer configured to obtain location information of the subject; and

a weather information obtainer configured to obtain at least one from among temperature, humidity, ultraviolet index, and fine dust concentration data corresponding to a location.

13. The apparatus of claim 10, wherein the lifestyle data comprises at least one from among an exercise time, and an outdoor activity time.

**Patentansprüche**

1. Verfahren zum Bereitstellen personalisierter Hautpflegeführungsinformationen, wobei das Verfahren Folgendes umfasst:

Erhalten von Hautgen-Expressionsdaten eines Subjekts;

Erhalten von externen Umgebungsdaten, Lifestyle-Daten des Subjekts und Hautzustandsindexdaten des Subjekts in Intervallen von einem ersten Intervall bis zu einem N-ten Intervall;

Analysieren einer Korrelation zwischen den Hautgen-Expressionsdaten, den externen Umgebungsdaten, den Lifestyle-Daten und den Hautzustandsindexdaten in den Intervallen von dem ersten Intervall zu dem N-ten Intervall;

Erhalten externer Umgebungsdaten, Lifestyle-Daten des Subjekts und Hautzustandsindexdaten des Subjekts in einem (N+1)-ten Intervall; und

Bereitstellen von Hautpflegeführungsinformationen des Subjekts an das Subjekt basierend auf einem Ergebnis der Korrelationsanalyse,

wobei die Lifestyle-Daten mindestens eines aus der Gruppe bestehend aus Schlafstunden, Alkoholkonsum, Hautpflege, Bewegungszeit, Outdoor-Aktivitätszeit und Menstruationszyklus beinhalten, wobei die Korrelation unter Verwendung der externen Umgebungsdaten und der Lifestyle-Daten als einer Eingabe und der Hautzustandsindexdaten als einer Ausgabe berechnet wird, und

wobei die Hautpflegeführungsinformationen des Subjekts eine Ursache für eine Änderung der Hautzustandsindexdaten SI des (N+1)-ten Tages basierend auf einem Ergebnis der Korrelationsanalyse zum N-ten Tag nach dem Erhalten externer Umgebungsdaten, Lifestyle-Daten und Hautzustandsindexdaten des (N+1)-ten Tages beinhalten, wobei das Analysieren der Korrelation das Trainieren eines künstlichen neuronalen Netzwerks unter Verwendung der externen Umgebungsdaten und der Lifestyle-Daten in den Intervallen von dem ersten Intervall zu dem N-ten Intervall, der Hautgen-Expressionsdaten als Eingabedaten für Training und der Hautzustandsindexdaten als Ausgabedaten für Training umfasst.

2. Verfahren nach Anspruch 1, wobei die externen Umgebungsdaten mindestens eines aus Temperatur-, Feuchte-, Ultraviolettindex-, Feinstaubkonzentrations- und Ultrafeinstaubkonzentrationsdaten umfassen.

3. Verfahren nach Anspruch 2, wobei das Erhalten der externen Umgebungsdaten Folgendes umfasst:

Erhalten von Standortinformationen des Subjekts; und

Erhalten von mindestens einem aus Temperatur-, Feuchte-, Ultraviolettindex-, Feinstaubkonzentrations- und Ultrafeinstaubkonzentrationsdaten, die einem Standort entsprechen.

4. Verfahren nach Anspruch 1, wobei die Lifestyle-Daten mindestens eines aus Bewegungszeit und Outdoor-Aktivitätszeit umfassen.

5. Verfahren nach Anspruch 1, wobei das Erhalten der Hautzustandsindexdaten das Berechnen eines Hautzustandsindex durch Analysieren einer Fotografie der Haut des Subjekts umfasst.

6. Verfahren nach Anspruch 1, wobei das Bereitstellen der Hautpflegeführungsinformationen an das Subjekt das Bereitstellen eines Alarms für das Subjekt umfasst, wenn eine Änderungsrate eines Hautzustandsindex des N-ten Intervalls und eines Hautzustandsindex des (N+1)-ten Intervalls gleich oder größer als ein Schwellenwert ist.

7. Verfahren nach Anspruch 1, wobei sowohl den externen Umgebungsdaten, den Lifestyle-Daten als auch den Hautzustandsindexdaten in der Korrelation eine Gewichtung zugewiesen wird und die Gewichtung auf einen größeren Wert eingestellt wird, wenn die Daten zu einer Zeit erhalten werden, die näher an der aktuellen Zeit ist.

8. Verfahren nach Anspruch 1, wobei die Hautpflegeführungsinformationen Leitinformationen über die Verwendung von Kosmetika und/oder einen Lebensstil und/oder eine Nährstoffaufnahme und/oder kosmetische Inhaltsstoffe umfassen.

9. Verfahren nach Anspruch 1, wobei das Bereitstellen der Hautpflegeführungsinformationen Folgendes umfasst:

Erhalten externer Umgebungsdaten eines (N+2)-ten Intervalls im (N+1)-ten Intervall; und
Bereitstellen von Hautpflegeführungsinformationen des (N+2)-ten Intervalls basierend auf dem Ergebnis der Korrelationsanalyse.

10. Vorrichtung zum Bereitstellen personalisierter Hautpflegeführungsinformationen, wobei die Vorrichtung Folgendes umfasst:

einen Datenerfasser, der dazu ausgelegt ist, Hautgen-Expressionsdaten eines Subjekts, externe Umgebungsdaten, Lifestyle-Daten eines Subjekts und Hautzustandsindexdaten des Subjekts in Intervallen von einem ersten Intervall bis zu einem N-ten Intervall zu erhalten;
einen Datenanalysator, der dazu ausgelegt ist, eine Korrelation zwischen Hautgen-Expressionsdaten, den externen Umgebungsdaten, den Lifestyle-Daten und den Hautzustandsindexdaten in den Intervallen von dem ersten Intervall zu dem N-ten Intervall zu analysieren; und
einen Hautpflegeführungsinformationsanbieter, der dazu ausgelegt ist, dem Subjekt basierend auf einem Ergebnis der Korrelationsanalyse Hautpflegeführungsinformationen bereitzustellen,
wobei der Datenanalysator durch Verwenden der Hautgen-Expressionsdaten, der externen Umgebungsdaten und der Lifestyle-Daten in den Intervallen von dem ersten Intervall zu dem N-ten Intervall als Eingabedaten für das Training und der Hautzustandsindexdaten als Ausgabedaten für das Training in einem künstlichen neuronalen Netzwerk trainiert wird,
der Datenerfasser ferner dazu ausgelegt ist, externe Umgebungsdaten, Lifestyle-Daten des Subjekts und Hautzustandsindexdaten des Subjekts in einem (N+1)-ten Intervall zu erhalten,
die Lifestyle-Daten mindestens eines aus Schlafstunden, Alkoholkonsum, Hautpflege, Bewegungszeit, Outdoor-Aktivitätszeit und Menstruationszyklus beinhalten,
die Korrelation unter Verwendung der externen Umgebungsdaten und der Lifestyle-Daten als Eingabe und der Hautzustandsindexdaten als Ausgabe berechnet wird, und
die Hautpflegeführungsinformationen des Subjekts eine Ursache für eine Änderung der Hautzustandsindexdaten SI des (N+1)-ten Tages basierend auf einem Ergebnis der Korrelationsanalyse zum N-ten Tag nach dem Erhalten externer Umgebungsdaten, Lifestyle-Daten und Hautzustandsindexdaten des (N+1)-ten Tages beinhalten.

11. Vorrichtung nach Anspruch 10, wobei die externen Umgebungsdaten mindestens eines aus Temperatur-, Feuchte-, Ultraviolettindex-, Feinstaubkonzentrations- und Ultrafeinstaubkonzentrationsdaten umfassen.

12. Vorrichtung nach Anspruch 10, wobei der Datenerfasser Folgendes umfasst:

einen Standortinformationserfasser, der dazu ausgelegt ist, Standortinformationen des Subjekts zu erhalten; und einen Wetterinformationserfasser, der dazu ausgelegt ist, mindestens eines aus Temperatur-, Feuchte-, Ultraviolettindex- und Feinstaubkonzentrationsdaten, die einem Standort entsprechen, zu erhalten.

**13.** Vorrichtung nach Anspruch 10, wobei die Lifestyle-Daten mindestens eines aus Bewegungszeit und Outdoor-Aktivitätszeit umfassen.


**Revendications**

**1.** Procédé permettant de fournir des informations personnalisées de guide de soins de la peau, le procédé comprenant les étapes suivantes :

obtenir des données d'expression génique de la peau d'un sujet ;
obtenir des données d'environnement extérieur, des données de mode de vie du sujet et des données d'indice d'état de la peau du sujet à des intervalles allant d'un premier intervalle à un N$^{ème}$ intervalle ;
analyser une corrélation entre les données d'expression génique de la peau, les données d'environnement extérieur, les données de mode de vie et les données d'indice d'état de la peau dans les intervalles allant du premier intervalle au N$^{ème}$ intervalle ;
obtenir des données d'environnement extérieur, des données de mode de vie du sujet et des données d'indice d'état de la peau du sujet dans un (N+1)$^{ème}$ intervalle ; et
fournir au sujet des informations de guide de soins de la peau du sujet, sur la base d'un résultat de l'analyse de corrélation,
les données de mode de vie comprennent au moins un élément parmi les heures de sommeil, la consommation d'alcool, les soins de la peau, le temps d'exercice, le temps d'activité en plein air et le cycle menstruel,
la corrélation étant calculée en utilisant les données d'environnement extérieur et les données de mode de vie en tant qu'entrée et les données d'indice d'état de la peau en tant que sortie, et
les informations de guide de soins de la peau du sujet comprennent une cause de changement dans les données d'indice d'état de la peau SI du (N+1)$^{ème}$ jour sur la base d'un résultat de l'analyse de corrélation avec le N$^{ème}$ jour après obtention des données d'environnement extérieur, des données de mode de vie, et des données d'indice d'état de la peau du (N+1)$^{ème}$ jour, moyennant quoi, l'analyse de la corrélation comprend d'effectuer l'apprentissage d'un réseau neuronal artificiel en utilisant les données d'environnement extérieur et les données de style de vie dans les intervalles allant du premier intervalle au N$^{ème}$ intervalle, les données d'expression génique de peau comme données d'entrée pour l'apprentissage et les données d'indice d'état de peau comme données de sortie pour l'apprentissage.

**2.** Procédé selon la revendication 1, dans lequel les données d'environnement extérieur comprennent au moins une donnée parmi la température, l'humidité, l'indice ultraviolet, la concentration en poussières fines et les données de concentration en poussières ultrafines.

**3.** Procédé selon la revendication 2, dans lequel l'obtention des données d'environnement extérieur comprend les étapes suivantes :

obtenir des informations d'emplacement du sujet ; et
obtenir au moins une donnée parmi la température, l'humidité, l'indice ultraviolet, la concentration en poussières fines et la concentration en poussières ultrafines correspondant à un emplacement.

**4.** Procédé selon la revendication 1, dans lequel les données de style de vie comprennent au moins un temps parmi un temps d'exercice et un temps d'activité en plein air.

**5.** Procédé selon la revendication 1, dans lequel l'obtention des données d'indice d'état de la peau comprend le calcul d'un indice d'état de la peau en analysant une photographie de la peau du sujet.

**6.** Procédé selon la revendication 1, dans lequel la fourniture des informations de guide de soins de la peau au sujet comprend la fourniture d'une alarme au sujet lorsqu'un taux de changement d'un indice d'état de peau du N$^{ème}$ intervalle et d'un indice d'état de peau du (N+1)$^{ème}$ intervalle est égal ou supérieur à une valeur de seuil.

**7.** Procédé selon la revendication 1, dans lequel un poids est attribué à chacune des données d'environnement extérieur, des données de mode de vie et des données d'indice d'état de la peau dans la corrélation, et le poids est fixé à une valeur plus grande lorsque les données obtenues à un instant plus proche de l'instant courant sont obtenues.

8. Procédé selon la revendication 1, dans lequel les informations de guide de soins de la peau comprennent au moins une information parmi des informations de guide concernant l'utilisation de cosmétiques, un mode de vie, l'apport nutritionnel et les ingrédients cosmétiques.

9. Procédé selon la revendication 1, dans lequel la fourniture des informations de guide de soins de la peau comprend les étapes suivantes :

obtenir des données d'environnement extérieur d'un (N+2)$^{\text{ème}}$ intervalle, dans le (N+1)$^{\text{ème}}$ intervalle ; et
fournir des informations de guide de soins de la peau pour le (N+2)$^{\text{ème}}$ intervalle, sur la base du résultat de l'analyse de corrélation.

10. Appareil permettant de fournir des informations personnalisées de guide de soins de la peau, l'appareil comprenant :

un dispositif d'obtention de données configuré pour obtenir des données d'expression génique de la peau d'un sujet, des données d'environnement extérieur, des données de mode de vie d'un sujet, et des données d'indice d'état de la peau du sujet à des intervalles allant d'un premier intervalle à un N$^{\text{ème}}$ intervalle ;
un analyseur de données configuré pour analyser une corrélation entre les données d'expression génique de la peau, les données d'environnement extérieur, les données de mode de vie et les données d'indice d'état de la peau dans les intervalles allant du premier intervalle au N$^{\text{ème}}$ intervalle ; et
un fournisseur d'informations de guide de soins de la peau configuré pour fournir des informations de guide de soins de la peau au sujet, sur la base d'un résultat de l'analyse de corrélation,
où l'analyseur de données effectue l'apprentissage d'un réseau neuronal artificiel en utilisant les données d'expression génique de la peau, les données d'environnement extérieur et les données de mode de vie dans les intervalles allant du premier intervalle au N$^{\text{ème}}$ intervalle comme données d'entrée pour l'apprentissage et les données d'indice d'état de la peau comme données de sortie pour l'apprentissage,
le dispositif d'obtention de données est en outre configuré pour obtenir en outre des données d'environnement extérieur, des données de mode de vie du sujet, et des données d'indice d'état de la peau du sujet dans un (N+1)$^{\text{ème}}$ intervalle,
les données de mode de vie comprennent au moins un élément parmi les heures de sommeil, la consommation d'alcool, les soins de la peau, un temps d'exercice, un temps d'activité en plein air et un cycle menstruel,
la corrélation est calculée à l'aide des données d'environnement extérieur et des données de style de vie en tant qu'entrée et des données d'indice d'état de la peau en tant que sortie, et
les informations de guide de soins de la peau du sujet comprennent une cause de changement dans les données d'indice d'état de la peau SI du (N+1)$^{\text{ème}}$ jour sur la base d'un résultat de l'analyse de corrélation avec le N$^{\text{ème}}$ jour après obtention des données d'environnement extérieur, des données de mode de vie et des données d'indice d'état de la peau du (N+1)$^{\text{ème}}$ jour.

11. Appareil selon la revendication 10, dans lequel les données d'environnement extérieur comprennent au moins une donnée parmi la température, l'humidité, l'indice ultraviolet, la concentration en poussières fines et les données de concentration en poussières ultrafines.

12. Appareil selon la revendication 10, dans lequel le dispositif d'obtention de données comprend :

un dispositif d'obtention d'informations de localisation configuré pour obtenir des informations de localisation du sujet ; et
un dispositif d'obtention d'informations météorologiques configuré pour obtenir au moins une donnée parmi la température, l'humidité, l'indice ultraviolet et la concentration en poussières fines correspondant à un emplacement.

13. Appareil selon la revendication 10, dans lequel les données de style de vie comprennent au moins un temps parmi un temps d'exercice et un temps d'activité en plein air.

# FIG. 1

# FIG. 2

START

OBTAIN EXTERNAL ENVIRONMENT DATA, LIFESTYLE DATA OF SUBJECT, AND SKIN CONDITION INDEX DATA IN INTERVALS FROM FIRST INTERVAL TO NTH INTERVAL — S21

ANALYZE CORRELATION AMONG EXTERNAL ENVIRONMENT DATA, LIFESTYLE DATA, AND SKIN CONDITION INDEX DATA IN INTERVALS FROM FIRST INTERVAL TO NTH INTERVAL — S22

OBTAIN EXTERNAL ENVIRONMENT DATA, LIFESTYLE DATA OF SUBJECT, AND SKIN CONDITION INDEX DATA IN (N+1)TH INTERVAL — S23

PROVIDE SKIN CARE GUIDE INFORMATION — S24

END

## FIG. 3

| ED₁ | ED₂ | EDₙ | ED_{N+1} |
|---|---|---|---|
| TEMPERATURE : 20°C<br>HUMIDITY : 25%<br>ULTRAVIOLET INDEX : 3<br>FINE DUST : 50μg/m³ | TEMPERATURE : 20°C<br>HUMIDITY : 25%<br>ULTRAVIOLET INDEX : 3<br>FINE DUST : 50μg/m³ | TEMPERATURE : 18°C<br>HUMIDITY : 50%<br>ULTRAVIOLET INDEX : 1<br>FINE DUST : 10μg/m³ | TEMPERATURE : 25°C<br>HUMIDITY : 20%<br>ULTRAVIOLET INDEX : 5<br>FINE DUST : 50μg/m³ |
| LD₁ | LD₂ | LDₙ | LD_{N+1} |
| SLEEPING HOURS : 6.5<br>DRINK : ×<br>SKIN CARE : ×<br>OUTDOOR ACTIVITY : 2 | SLEEPING HOURS : 7<br>DRINK : ○<br>SKIN CARE : ×<br>OUTDOOR ACTIVITY : 3 | SLEEPING HOURS : 8<br>DRINK : ×<br>SKIN CARE : ×<br>OUTDOOR ACTIVITY : 1 | SLEEPING HOURS : 6<br>DRINK : ×<br>SKIN CARE : ×<br>OUTDOOR ACTIVITY : 0 |

FIRST INTERVAL  SECOND INTERVAL  · · ·  NTH INTERVAL  (N+1)TH INTERVAL  S23

S22

ANALYZE CORRELATION

P₁    P₂    Pₙ    P_{N+1}

SI₁ — 83    SI₂ — 72    · · ·    SIₙ — 95    SI_{N+1} — 82

S21

S24

PROVIDE SKIN CARE GUIDE INFORMATION

# FIG. 4

ED N2

TEMPERATURE : 20°C
HUMIDITY : 25%
ULTRAVIOLET INDEX : 3
ULTRAVIOLET INDEX : 50μg/m³

ED N1

ED N3

HOUSE —————— GOING OUT —————— HOUSE

t₁          t₂          t₃

GWANAK-GU, SEOUL(41)

GWANAK-GU, SEOUL(42)

GWANAK-GU, SEOUL(43)

# FIG. 5

10

WRINKLES

PIGMENT

51

TROUBLE

WRINKLES : 82
PIGMENT : 80
TROUBLE: 76

52

# FIG. 6

# FIG. 7

FINE DUST
CONCENTRATION

0  1  2  . . . . .  N  N+1  TIME

ULTRAVIOLET
INDEX

0  1  2  . . . . .  N  N+1  TIME

PIGMENT INDEX

0  1  2  . . . . .  N  N+1  TIME

# FIG. 8

PIGMENT INDEX

TIME

0  1  2  . . . . .  N  N+1

20% REDUCTION

20% REDUCTION IN PIGMENT INDEX! ——— 81

• TODAY, YOU'VE HAD TOO MUCH UV! ——— 82

SLEEP AFTER TAKING VITAMIN C! ——— 83

10

# FIG. 9

$ED_{N+2}$   FINE DUST CONCENTRATION: HIGH
ULTRAVIOLET INDEX: HIGH

CURRENT TIME : N+1

10

ULTRAVIOLET INDEX IS EXPECTED TO BE HIGH TOMORROW, SO SUNSCREEN IS ESSENTIAL BEFORE GOING OUT! ——— 91

UV PROTECTION

SPF 50 PA+++ ——— 92

# FIG. 10

OBTAIN SKIN GENE EXPRESSION DATA — S100

START

OBTAIN EXTERNAL ENVIRONMENT DATA, LIFESTYLE DATA OF SUJBECT, AND SKIN CONDITION INDEX DATA IN INTERVALS FROM FIRST INTERVAL TO NTH INTERVAL — S101

ANALYZE CORRELATION AMONG EXTERNAL ENVIRONMENT DATA, LIFESTYLE DATA, AND SKIN CONDITION INDEX DATA IN INTERVALS FROM FIRST INTERVAL TO NTH INTERVAL — S102

OBTAIN EXTERNAL ENVIRONMENT DATA, LIFESTYLE DATA OF SUBJECT, AND SKIN CONDITION INDEX DATA IN (N+1)TH INTERVAL — S103

PROVIDE SKIN CARE GUIDE INFORMATION — S104

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20150105574
- KR 20140075258

- US 2010185064 A1